# EUROPEAN PATENT SPECIFICATION

(11) **EP 4 333 721 B1**
(45) Date of publication and mention of the grant of the patent: **17.12.2025**
(21) Application number: 21939683.5
(22) Date of filing: 07.05.2021
(51) Int. Cl.: A61B 17/072

(54) **AUXILIARY DEVICES FOR STAPLING APPARATUS**
HILFSVORRICHTUNGEN FÜR EINE KLAMMERVORRICHTUNG
DISPOSITIFS AUXILIAIRES POUR APPAREIL D'AGRAFAGE

(43) Date of publication of application: 13.03.2024
(73) Proprietor: Covidien LP, Mansfield, MA 02048 (US)
(72) Inventor: JIA, Yingwei, Shanghai 201114 (CN)
(74) Representative: Maschio & Soames IP Ltd
(86) International application number: PCT/CN2021/092162
(87) International publication number: WO 2022/233036

(56) References cited:
- WO-A1-2015/179783
- WO-A1-2019/169010
- CN-A- 110 025 348
- CN-A- 110 074 833
- CN-A- 111 820 971
- CN-U- 208 524 936
- JP-A- 2010 000 372
- US-A- 2 892 457
- US-A1- 2009 206 124
- US-A1- 2015 209 821
- US-A1- 2017 290 583
- US-B2- 10 631 866

## Description

### FIELD

The disclosure relates to surgical stapling, and more particularly, to auxiliary devices for stapling apparatus that facilitate firing and positioning of the stapling apparatus during a surgical procedure.

### BACKGROUND

Stapling apparatus are employed by clinicians to apply one or more rows of staples, sequentially or simultaneously, to body tissue for the purpose of anastomosing tissue segments of body tissue. These stapling apparatuses generally include an anvil and a staple cartridge that are movable in relation to each other between unclamped and clamped positions to clamp tissue. When the stapling apparatus is actuated, or "fired," longitudinally moving firing bars or cams contact staple pushers located within the staple cartridge to eject staples from the staple cartridge.

Stapling apparatus, such as open linear staplers, are widely used for gastric and intestinal anastomosis and include a firing knob that is movable to simultaneously eject staples from the staple cartridge of the stapling apparatus and to cut tissue clamped between the staple cartridge and the anvil of the stapling apparatus. The force required to advance the firing knob through a firing stroke may be high.

During gastrointestinal surgeries, multiple firing strokes are generally required during a single surgical procedure. Such effort may cause fatigue in the hands of a clinician. This fatigue can result in incomplete firing and/or involuntary muscle movements and it may be exacerbated during surgical procedures in which thick tissue, e.g., stomach or bronchus tissue is treated. Incomplete firing may cause bleeding and anastomotic leakage and involuntary muscle movements in the hands of a clinician may cause unintentional handshakes that may result in pulling of the tissue.

In some cases, a clinician may be required to hold the stapling apparatus at awkward angles to perform a surgical procedure. Achieving the required firing force from such awkward angles may further worsen the problems described above.

Furthermore certain auxiliary devices for syringes are known from documents US2015/209821 A1, WO2015/179783 A1 and US 2892457 A.

### SUMMARY

The techniques of this disclosure generally relate to an auxiliary device that supports a surgical stapling apparatus in a variety of orientations and facilitates firing and retracting movements of a firing knob of the surgical stapling apparatus.

According to aspects of the disclosure, an auxiliary device for a surgical stapler includes a body, a mounting bracket, a drive plate, a rack, and a firing lever. The mounting bracket is supported on the body and configured to releasably couple the body to the surgical stapler. The drive plate is configured to couple to a firing actuator of the surgical stapler. The rack is mechanically coupled to the drive plate. The firing lever is mechanically coupled to the rack and pivotable to move the drive plate relative to the surgical stapler for firing the surgical stapler.

In aspects, the mounting bracket may be rotatably coupled to the body. The body may define a longitudinal axis. The mounting bracket may be positioned to rotate in a clockwise direction up to about 90 degrees about the longitudinal axis. The mounting bracket may be positioned to rotate in a counterclockwise direction up to about 90 degrees about the longitudinal axis.

In aspects, the drive plate may define a slot configured to receive the firing actuator. The slot may be configured to slidably support the firing actuator as the surgical stapler is rotated about the auxiliary device.

In aspects, the drive plate may be movable relative to the body.

In aspects, the auxiliary device may include a stationary handle. The firing lever may be movable relative to the stationary handle.

In aspect the auxiliary device may include a pawl. The rack may include a plurality of teeth. The pawl may be configured to mechanically engage with the plurality of teeth to move the rack relative to the body as the firing lever moves relative to the stationary handle.

In aspects, the drive plate may include a retracting handle, which when pulled, may draw the drive plate toward an unfired position.

In aspects, the drive plate may have an arcuate shape configured to enable the surgical stapler to rotate about the drive plate.

According to the invention and as defined in claim 1, a surgical stapling system includes a surgical stapler and an auxiliary device. The surgical stapler includes an end effector and a firing actuator configured to fire the end effector. The auxiliary device includes a mounting bracket, a drive plate, a rack, and a firing lever. The mounting bracket is releasably coupled to the surgical stapler. The drive plate is coupled to the firing actuator of the surgical stapler. The rack is mechanically coupled to the drive plate. The firing lever is mechanically coupled to the rack and pivotable to move the drive plate relative to the surgical stapler for firing the surgical stapler.

In aspects, the auxiliary device may define a longitudinal axis. The mounting bracket may be positioned to rotate about the longitudinal axis to enable the surgical stapler to rotate about the drive plate.

In aspects, the mounting bracket may be positioned to rotate in a clockwise direction up to about 90 degrees about the longitudinal axis, and wherein the mounting bracket may be positioned to rotate in a counterclockwise direction up to about 90 degrees about the longitudinal axis.

In aspects, the drive plate may define a slot in which the firing actuator is received. The slot may be configured to slidably support the firing actuator as the surgical stapler is rotated about the auxiliary device.

In aspects, the drive plate may be axially movable along the longitudinal axis.

In aspects, the auxiliary device may further include a stationary handle. The firing lever may be movable relative to the stationary handle.

In aspects, the surgical stapling system may further include a pawl, and wherein the rack may include a plurality of teeth. The pawl may be configured to mechanically engage with the plurality of teeth to move the rack relative to the stationary handle as the firing lever moves relative to the stationary handle.

In aspects, the drive plate may include a retracting handle, which when pulled, may draw the drive plate toward an unfired position.

According to yet another aspect, this disclosure is directed to an auxiliary device for a surgical stapler. The auxiliary device includes a body, a mounting bracket, a drive plate, and a firing lever. The mounting bracket is supported on the body and configured to releasably couple the body to the surgical stapler. The drive plate is configured to releasably couple to a firing actuator of the surgical stapler. The firing lever is supported on the body and pivotable to move the drive plate relative to the surgical stapler for firing the surgical stapler when the surgical stapler is releasably coupled to the auxiliary device.

The details of one or more aspects of the disclosure are set forth in the accompanying drawings and the description below. Other features, objects, and advantages of the techniques described in this disclosure will be apparent from the description and drawings, and from the claims that follow.

### BRIEF DESCRIPTION OF THE DRAWINGS

Various aspects of this disclosure are described herein with reference to the drawings, wherein:
FIG. 1A is a perspective view of a stapling apparatus in a clamped, pre-fired position;
FIG. 1B is a side, perspective view of the stapling apparatus of FIG. 1A in an open position;
FIG. 2 is a perspective view of an auxiliary device for the stapling apparatus of FIGS. 1A and 1B according to aspects of the disclosure;
FIG. 3 is an exploded, perspective view of the auxiliary device of FIG. 2;
FIG. 4 is an enlarged, side view illustrating a ratchet-rack mechanism of the auxiliary device of FIG. 2;
FIGS. 5A-5C are progressive views illustrating the stapling apparatus of FIGS. 1A and 1B being secured to the auxiliary device of FIG. 2;
FIG. 6A is a side view illustrating the auxiliary device of FIG. 2 shown coupled to the stapling apparatus of FIGS. 1A and 1B;
FIG. 6B is an enlarged, cross-sectional view taken along section line C-C of FIG. 6A;
FIG. 6C is an enlarged, cross-sectional view taken along section line D-D of FIG. 6A;
FIG. 6D is a perspective view illustrating the stapling apparatus of FIGS. 1A and 1B rotated to a first position relative to the auxiliary device of FIG. 2; and
FIGS. 7A-7D are progressive cross-sectional views illustrating a firing process of the auxiliary device.

### DETAILED DESCRIPTION

Aspects of the disclosure will now be described in detail with reference to the drawing figures wherein like reference numerals identify similar or identical elements. Throughout this description, the term "proximal" refers to a portion of a structure, or component thereof, that is closer to a user, and the term "distal" refers to a portion of the structure, or component thereof, that is farther from the user. As commonly known, the term "clinician" refers to a doctor, a nurse, or any other care provider and may include support personnel. In addition, directional terms such as left, right and the like are used simply for convenience of description and are not intended to limit the disclosure attached hereto.

In the following description, well-known functions or constructions are not described in detail to avoid obscuring the present disclosure in unnecessary detail.

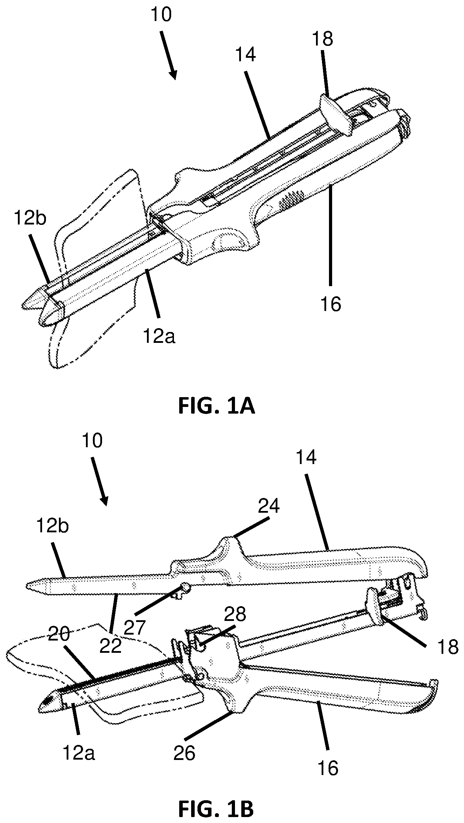

FIGS. 1A and 1B illustrate a surgical stapling apparatus in the form of an open linear stapler, although any suitable surgical stapling apparatus may be provided (e.g., endoscopic, transverse, circular, etc.). The surgical stapling apparatus is shown as stapling device 10 and generally includes a cartridge half section 12a, an anvil half section 12b, a clamping lever 16, and a firing knob or a firing actuator 18. The cartridge half-section 12a includes a distal portion that supports a staple cartridge 20 and a proximal portion that pivotably supports the clamping lever 16. The clamping lever 16 is engaged with the anvil half-section 12b and is pivotable from a first position (FIG. 1B) to a second position (FIG. 1A) to move the stapling device 10 from an unclamped position to a clamped position. In the clamped position, the firing actuator 18 is aligned with the cartridge half-section 12a and forms part of a grip of the stapling device 10. The anvil half-section 12b includes a distal portion that supports an anvil plate 22 and a proximal portion that is pivotably engaged with the proximal portion of the cartridge half-section 12a and includes a lever handle 14.

The anvil half-section 12b of stapling device 10 has a central portion that includes a pair of cylindrical lateral support members 27. During assembly of the anvil half-section 12b and the cartridge half-section 12a, the lateral support members 27 are supported in U-shaped recesses or cutouts 28 defined in a distal portion of the clamping lever 16 such that when the clamping lever 16 is moved to the second or clamped position (FIG. 1A), the anvil half-section 12b is pivoted towards the cartridge half-section 12a. The cutouts 28 are shaped to receive and hold the lateral support members 27 of the anvil half-section 12b to retain the anvil half-section 12b and the cartridge half-section 12a in the clamped position when the clamping lever 16 is moved from the first or unclamped position (FIG. 1B) to the second or clamped position (FIG. 1A).

To position the stapling device 10 in the clamped position, the clamping lever 16 is rotated in a counterclockwise direction from the position shown in FIG. 1B after the anvil half-section 12b and the cartridge half-section 12a are attached. As the clamping lever 16 is rotated toward the cartridge half-section 12a, the lateral support members 27 are received in the cutouts 302 of the clamping lever 16 and are cammed towards the cartridge half-section 12a.

The firing actuator 18 of the stapling device 10 is supported on the cartridge half-section 12a for movement between retracted and advanced positions to actuate ejection of staples from the staple cartridge 20. When the clamping lever 16 is moved towards the lever handle 14, the anvil half-section 12a and the cartridge half-section 12b are urged towards each other to clamp tissue to be treated. With the stapling device 10 in the clamped position, the firing actuator 18 may be operated (e.g., moved distally from a retracted position towards an advanced position) to sequentially fire and form a plurality of surgical staples of the staple cartridge 20 within the tissue clamped between the cartridge half-section 12a and the anvil half-section 12b.

The anvil plate 22 of the anvil half-section 12b may include a plurality of staple forming pockets or cavities (not shown) defined in an inwardly or tissue facing surface thereof. Staples (not shown) that are ejected from the staple cartridge 20 are received and formed in the staple forming pockets of the anvil plate 22.

When a clinician pushes the firing actuator 18 toward the distal portions of the anvil and cartridge half-sections 12b, 12a, staple firing is activated. In this regard, the clamping lever 16 and the proximal portion of the anvil half-section 12b include protrusions 24, 26 which are engaged by a clinician's hand or finger (e.g., the thumb) so that the clinician can firmly grasp the stapling device 10 while the firing actuator 18 is pushed through a firing stroke. For a more detailed description of the structure and operation of a similar stapling device, one or more components of which are included or can be modified for use with the disclosed stapling apparatus 10, reference can be made to U.S. Patent No. 9,724,095.

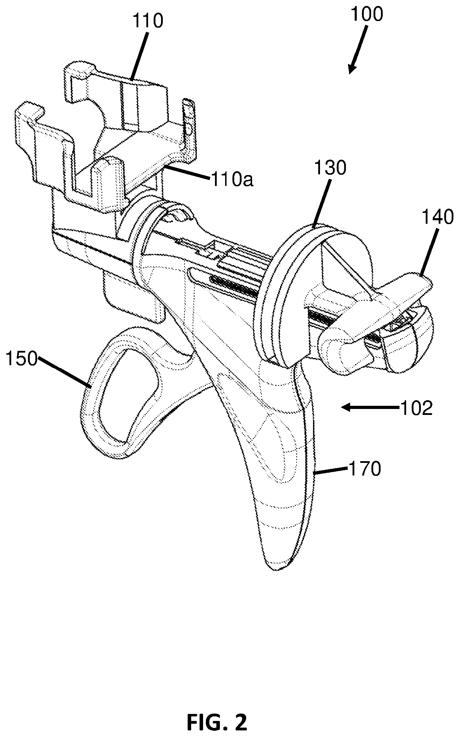

FIG. 2 illustrates an auxiliary device 100 which helps a clinician position and fire the stapling device 10 when the auxiliary device 100 is coupled thereto. The stapling device 10 can be supported on the auxiliary device 100 to reduce an amount of force required by a clinician to fire staples through mechanical advantage and to position the stapling device 10 in an advantageous orientation relative to a surgical site that helps the clinician easily and conveniently operate the stapling device 10 during a surgical procedure at the surgical site. The auxiliary device 100 also enables clinicians to comfortably hold and fire the stapling device 10 in a more controlled manner.

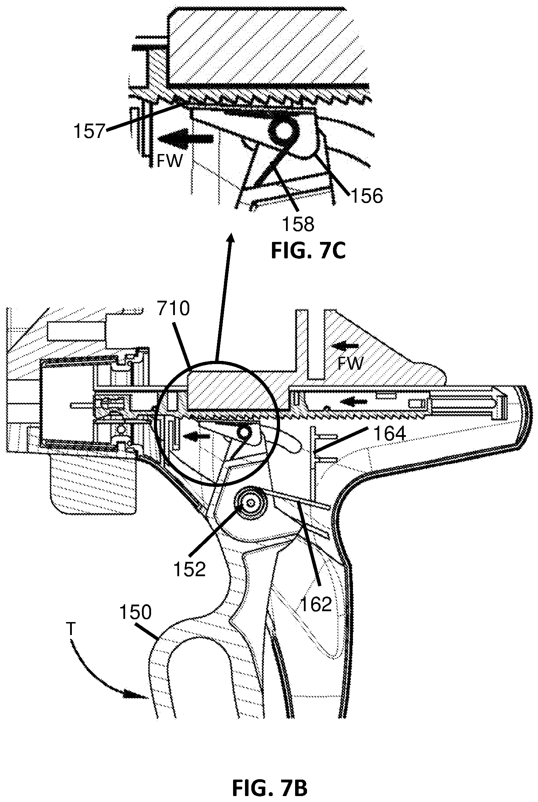

With reference to FIGS. 2-4, the auxiliary device 100 defines a longitudinal axis "L" and includes a mounting bracket 110, a drive plate 130, a retraction handle 140, a firing lever 150, and a stationary handle 170 that provides a gripping surface for enabling a clinician to grip the auxiliary device 10. The mounting bracket 110 selectively supports and retains the stapling device 10 of FIGS. 1A and 1B. The drive plate 130 includes two side walls 130a, which define a slot 131 between the two side walls 130a for receiving and coupling to the firing actuator 18 of the stapling device 10. The firing lever 150 of the auxiliary device 100 is coupled to the drive plate 130 to enable the drive plate 130 to longitudinally translate the firing actuator 18 of the stapling device 10 between a retracted or proximal position and an advanced or distal position to fire the stapling device 10. In an aspect of the disclosure, a ratchet-rack mechanism (e.g., 710 of FIG. 7B) of the auxiliary device 100 is couples the firing lever 150 and the drive plate 130 to facilitate the linear movement of the firing actuator 18 of the stapling device 10.

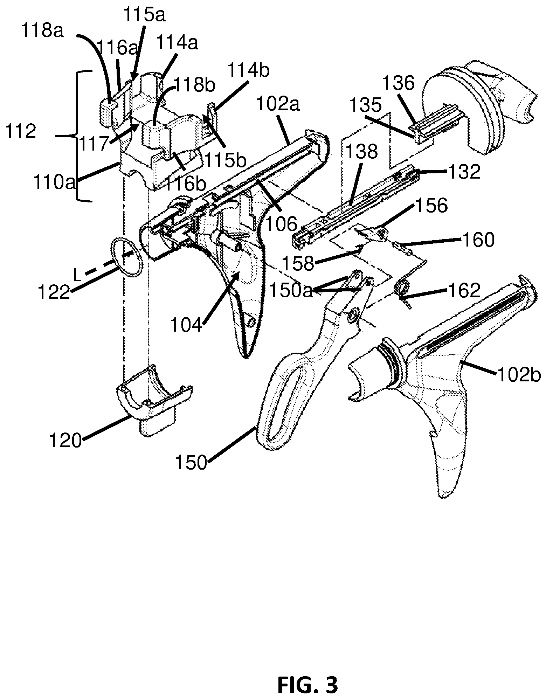

FIG. 3 illustrates an exploded view of the auxiliary device 100 of FIG. 2. The mounting bracket 110 includes an upper portion 112, a lower portion 120, and a washer 122. The upper and lower portions 112, 120 of the mounting bracket 110 are secured about a distal part of a body 102 of the auxiliary device 100. The body 102 includes first and second halves 102a, 102b. The washer 122, the mounting bracket 110, and the distal portion of the first and second halves 102a, 102b of body 102 enable bi-rotational (e.g., clockwise and counterclockwise) movements of the mounting bracket 110 around the longitudinal axis "L" of the auxiliary device 100.

The upper portion 112 of the mounting bracket 110 includes a base 110a and four protrusions, 114a, 114b, 116a, 116b, that extend from the base 110a. The upper portion 112 further includes two rear mounting bracket walls 114a, 114b and two front mounting bracket walls 116a, 116b. The rear mounting bracket wall 114a and the front mounting bracket wall 116a define a left-side slot 115a, and the rear mounting bracket wall 114b and the front mounting bracket wall 116b define a right-side slot 115b. These two slots 115a, 115b receive the protrusions 24, 26 (FIG. 1B) of the lever handle 14 and the clamping lever 16, respectively, of the stapling device 10 to prevent forward and backward (e.g., proximal and distal) movements of the stapling device 10 relative to the auxiliary device 100 and along the longitudinal axis "L" of the auxiliary device 100.

The front mounting bracket walls 116a and 116b of the upper portion 112 define a front slot 117, which receives the anvil half section 12a and the cartridge half section 12b (FIG. 1B) of the stapling device 10. Two retaining protrusions 118a and 118b of upper portion 112 protrude from the top surface of the front mounting bracket walls 116a and 116b in a direction substantially parallel to the longitudinal axis "L" of the auxiliary device 100. When the anvil half section 12a and the cartridge half section 12b of the stapling device 10 are inserted through the front slot 117 of upper portion 112, the two retaining protrusions 118a and 118b secure the stapling device 10 to mounting bracket 110 of auxiliary device 100.

The first and second halves 102a and 102b of the body 102 of the auxiliary device 100 are secured together using known fastening devices or techniques to define a cavity 104 in the body 102. A receiving base 106 is formed within the cavity 104 to receive a rack 132 that is configured to receive a mating part 136 of the drive plate 130. A tap or rib 135 on the bottom of the mating part 136 matingly fits into an elongated slot 138 defined in the rack 132.

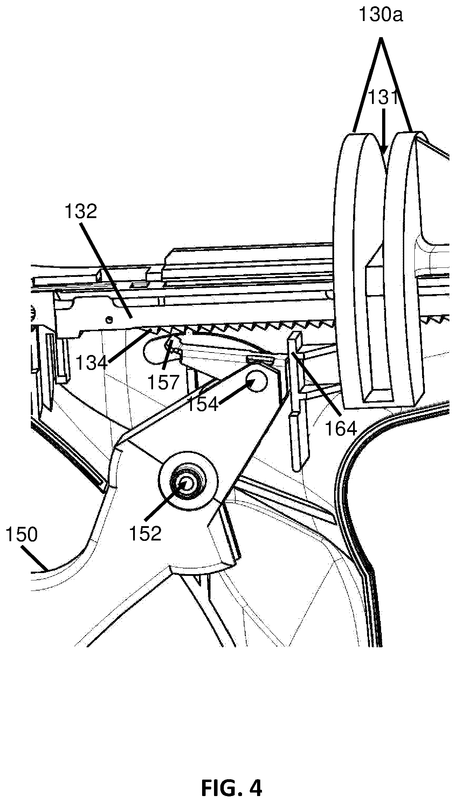

The firing lever 150 of auxiliary device 100 is supported on the body 102 of the auxiliary device 100 and rotates around a pivot 152 (FIG. 4) between an initial or unfired position and a fired position. The firing lever 150 may pivot back and forth between the unfired position and the fired position one or more times to fire staples from the staple cartridge 20 (FIG. 1B). A spring 162 of the auxiliary device 100 provides an elastic force so that the firing lever 150 automatically returns (e.g., is biased or urged) to the unfired position from the fired position.

The top portion of the firing lever 150 includes a pair of side walls 150a, each of which has a hole 154 (FIG. 4). A ratchet member 156 is disposed and fastened between the two side walls via a pin 160. Further, a pawl spring 158 is positioned with the ratchet member 156 via the pin 160. The ratchet member 156, the firing lever 150, and the rack 132 form a ratcheting mechanism (e.g., 710 of FIG. 7B), which enables movement of the drive plate 130 toward the distal part of the auxiliary device 100 so that the firing actuator 18 of the stapling device 10 is moved, with mechanical advantage, from the retracted position to the advanced position in response to movement of the firing lever 150 from the initial position towards the fired position for firing staples from the staple cartridge 20 (FIG. 1B).

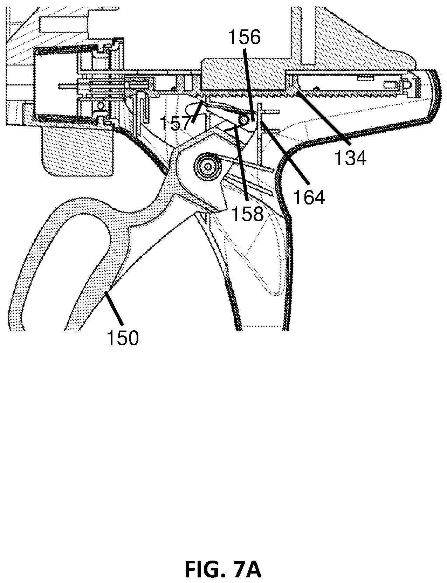

As best seen in FIG. 3, 4, 7A and 7B, ratcheting mechanism 710 of the auxiliary device 100 includes a locking pawl 157 that is fixedly attached to the top surface of or a portion of the ratchet member 156, and gear teeth 134 that depend from the rack 132. When the firing lever 150 is positioned in the initial, unfired position, a back surface of the ratchet member 156 engages a support wall 164 formed within the cavity 104 of the body 102 of the auxiliary device 100. Due to the configuration of the ratchet member 156, the locking pawl 157 is detached or separated from the gear teeth 134 when the ratchet member 156 touches the support wall 164. When the firing lever 150 is in the initial, unfired position, the locking pawl 157 is detached from the gear teeth 134. The ratcheting mechanism 710, which moves the rack 132 toward the fired position, is described in greater detail below with reference to FIGS. 7A-7D.

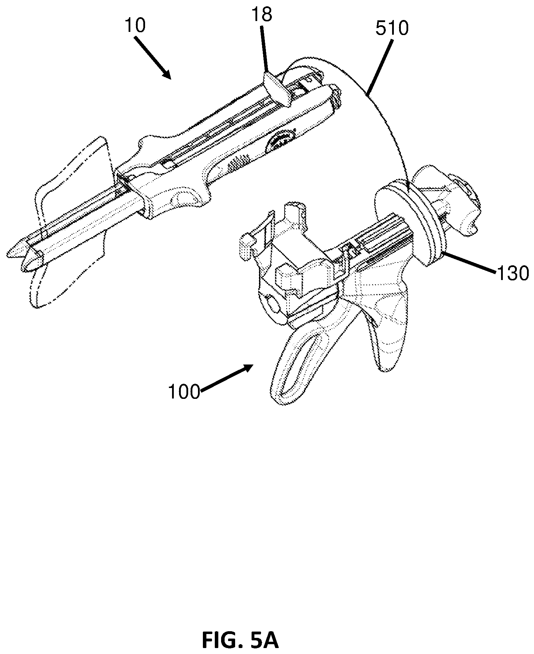

FIGS. 5A-5C illustrate how the stapling device 10 is attached to the auxiliary device 100. Firstly, the stapling device 10 is positioned such that the firing actuator 18 faces downward so that the firing actuator 18 can fit within the slot 131 (FIG. 4) of the drive plate 130 of the auxiliary device 100, as indicated by an arrow 510 in FIG. 5A.

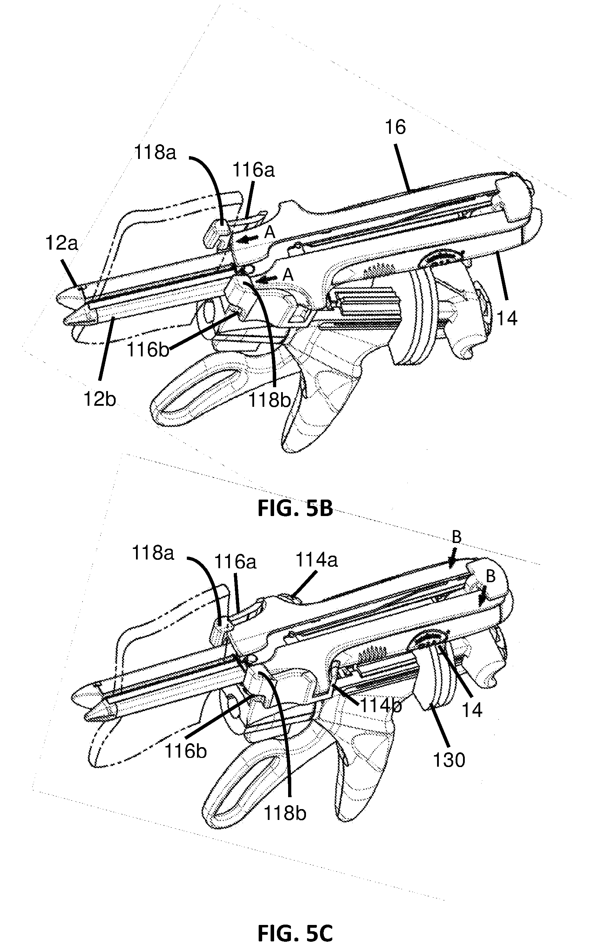

Secondly, as illustrated in FIG. 5B, the anvil half section 12a and the cartridge half section 12b of the stapling device 10 are inserted into the front slot 117 (FIG. 3) of the upper portion 112 of the mounting bracket 110 at an inclined orientation in the direction of arrows "A" so that the protrusions 24, 26 (FIG. 1B) of the lever handle 14 and the clamping lever 16, respectively, can be partly inserted into or positioned over the side slots 115a, 115b (FIG. 3) defined between the rear mounting bracket walls 114a, 114b and the front mounting bracket walls 116a, 116b, respectively, of the upper portion 112 of the mounting bracket 110.

Thirdly, as illustrated in FIG. 5C, the proximal portion of the stapling device 10 is pushed down in the direction of arrows "B" so that the protrusions 24, 26 of the lever handle 14 and the clamping lever 16, respectfully, are fully inserted into the side slots 115a, 115b (FIG. 3) of the upper portion 112 of the mounting bracket 110 and the firing actuator 18 (FIG. 1A) is inserted into the slot 131 (FIG. 4) of the drive plate 130. By doing so, the distal portion of the lever handle 14 and the clamping lever 16 is locked/retained by the retaining protrusions 118a and 118b (FIG. 3) of upper portion 112. This causes the stapling device 10 to be retained or locked within the mounting bracket 110 of the auxiliary device 100.

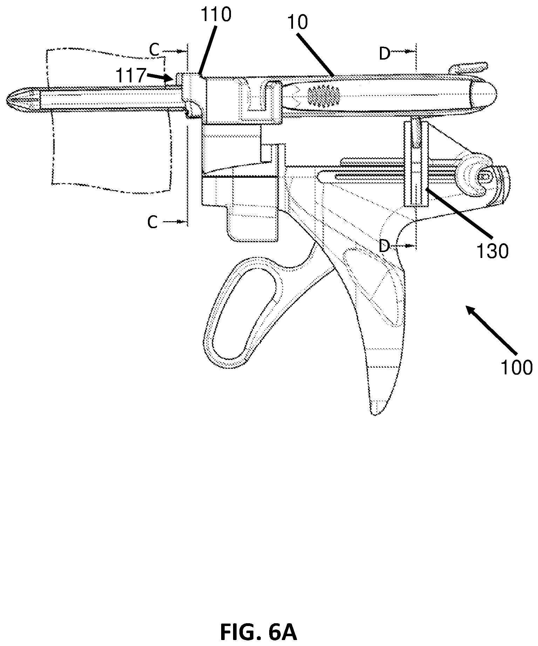

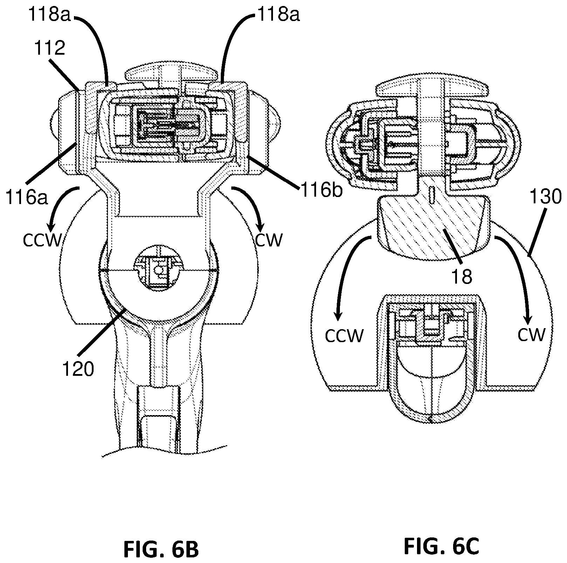

FIGS. 6A-6C illustrate views of the stapling device 10 retained within the auxiliary device 100. FIG. 6B illustrates that the distal portions of the stapling device 10 are slidably inserted within the front slot 117 defined between the front mounting bracket walls 116a, 116b of the upper portion 112 of the mounting bracket 110 and retained by the retaining protrusions 118a and 118b. The assembly of the upper and lower portions 112, 120 of the mounting bracket 110 enables the mounting bracket 110 to rotate around the longitudinal axis "L" of the auxiliary device 100 counterclockwise or clockwise, as indicated by arrows "CCW" and "CW," respectively. In aspects, the mounting bracket 110 may be capable of rotating up to about 90° in clockwise and/or counterclockwise directions about the longitudinal axis "L". This rotation enables the relative positions of the stapling device 10 and the auxiliary device 100 to be changed to adapt to different firing positions and/or different routines of various clinicians so that a variety of different surgical procedures can be performed more effectively and efficiently while reducing uncomfortable or awkward firing positions.

FIG. 6C illustrates the firing actuator 18 inserted into the slot 131 formed by two side walls 130a (FIG. 4) of drive plate 130. For example, when the mounting bracket 110 is rotated counterclockwise as indicated by arrows "CCW," the stapling device 10 is correspondingly rotated and the firing actuator 18 slides through the slot 131 of the drive plate 130. While the stapling device 10 is retained or locked within the mounting bracket 110 of the auxiliary device 100, the firing actuator 18 is also retained or locked within the slot 131 of the drive plate 130.

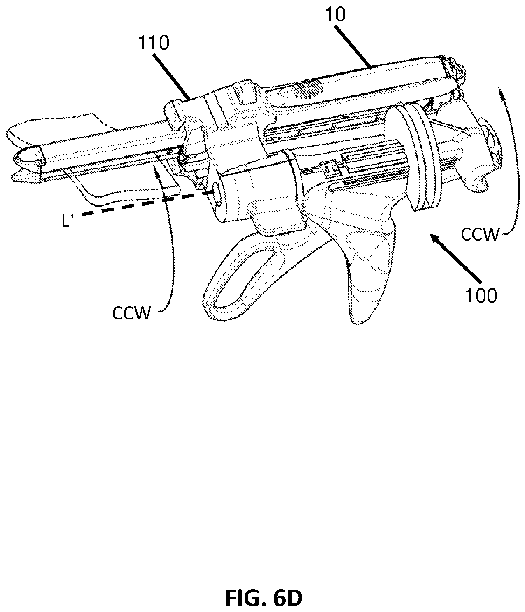

FIG. 6D illustrates a perspective view of the mounting bracket 110 of the auxiliary device 110 and the stapling device 10 as the mounting bracket 110 is rotated 90 degrees to the right of auxiliary device 110. In aspects of the disclosure, the mounting bracket 110 may be configured to rotate by a unit angle for locking the mounting bracket 110 at predetermined angular positions in relation to the body 102 of the auxiliary device 100 (e.g., through angular intervals that may be audibly or haptically indicated via a click, for instance). The unit angles, for example, may be 1, 2, 5, 10, 20, 30 degrees, etc., in clockwise and/or counterclockwise directions about the longitudinal axis "L" of the auxiliary device 100.

FIGS. 7A-7D illustrate operation of the firing lever 150 and ratchet member 156 to advance the rack 132 and fire the stapling device 10. In FIG. 7A, the firing lever 150 is in the initial, unfired position where the back surface of the ratchet member 156 touches the support wall 164 of the body 102 of the auxiliary device 100. Based on the shape of the back surface of the ratchet member 156, which touches the support wall 164 of the body 102 of the auxiliary device 100, the locking pawl 157 is not engaged with the gear teeth 134 of the rack 132. The downward force exerted by the support wall 164 on the ratchet member 156, when the firing lever 150 is in the initial, unfired position, is greater than the elastic force exerted toward the gear teeth 134 by the pawl spring 158 so that the locking pawl 157 is disengaged from the gear teeth 134 in the initial, unfired position. Further, the elastic force of the spring 162 urging the firing lever 150 to the initial, unfired position is greater than the elastic force of the pawl spring 158 that urges the ratchet member 156 towards the rack 132. Thus, without an external force applied to the firing lever 150 by a clinician, the firing lever 150 is maintained in the initial, unfired position.

FIGS. 7B and 7C illustrate the firing lever 150 in or near the fired position, where the ratchet member 156 is separated from the support wall 164. In this position, the locking pawl 157 is engaged with the gear teeth 134 of the rack 132 of the ratcheting mechanism 710. Without the downward force on the ratchet member 156 applied by the support wall 164, the locking pawl 157 is urged toward the gear teeth 134 by the elastic force from the pawl spring 158. Thus, after the locking pawl 157 engages the gear teeth 134, the rotational movement of the firing lever 150 around the pivot 152 is translated into a forward linear movement, "FW," of the rack 132 toward the firing position. Since the rack 132 is fixedly attached to the drive plate 130 of the auxiliary device 100, forward movements of the rack 132 cause forward movements of the drive plate 130.

In aspects of the disclosure, one stroke "T" of the firing trigger from the initial, unfired position to the fired position might not be sufficient to move the rack 132 to a firing or fully advanced position for fully actuating the stapling device 10. In this case, one or more strokes of the firing lever 150 may be needed to fire all of the staples from the staple cartridge 20 of the stapling device 10. The elastic force exerted by the spring 162 may enable the firing lever 150 to automatically move from the fired position to the initial, unfired position. During this movement to the initial, unfired position, the locking pawl 157 smoothly, slidably moves backward based on the saw-tooth shape of the gear teeth 134 so that the rack 132 does not move back but stays at an advanced position.

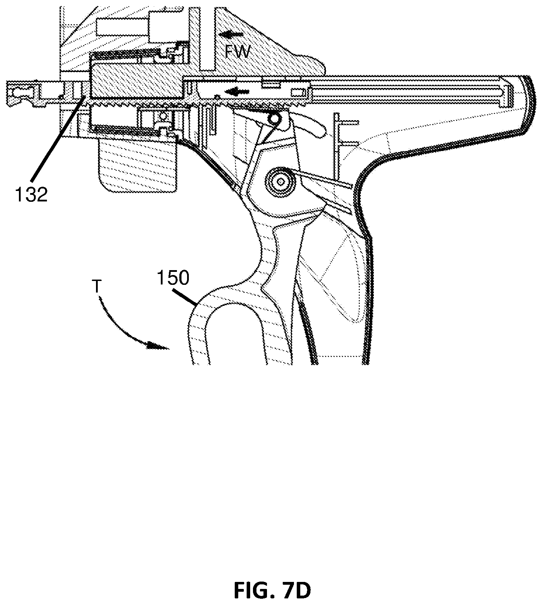

After one or more strokes of the firing lever 150, the rack 132 arrives at the fully advanced and fired position in which all of the staples are fired from the stapling device 10 as illustrated in FIG. 7D. After completion of firing of the stapling device 10, the retraction handle 140 (FIG. 2) can be pulled to draw the drive plate 130 proximally from the fired position so that the stapling device 10 can be returned to the initial, unfired position. In this position, the stapling device 10 can be removed from the surgical site by the clinician's removal of the auxiliary device 100 or can be ready for firing more staples.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques).

While several aspects of the disclosure have been shown in the drawings, it is not intended that the disclosure be limited thereto, as it is intended that the disclosure be as broad in scope as the art will allow and that the specification be read likewise. It is to be understood, therefore, that the disclosure is not limited to the precise aspects described, and that various other changes and modifications may be affected by one skilled in the art without departing from the scope or spirit of the disclosure. Additionally, the elements and features shown and described in connection with certain aspects may be combined with the elements and features of certain other aspects without departing from the scope of the disclosure, and that such modifications and variation are also included within the scope of the disclosure. Therefore, the above description should not be construed as limiting, but merely as exemplifications of aspects of the disclosure. Thus, the scope of the aspects of the invention should be determined by the appended claims and their legal equivalents.

## Claims

1. A surgical stapling system comprising:
a surgical stapler (10) including an end effector and a firing actuator (18) configured to fire the end effector;
**characterised in that** the surgical stapling system further comprises an auxiliary device (100) including:
a mounting bracket (110) releasably coupled to the surgical stapler (10);
a drive plate (130) coupled to the firing actuator (18) of the surgical stapler (10);
a rack (132) mechanically coupled to the drive plate (130); and
a firing lever (150) mechanically coupled to the rack (132) and pivotable to move the drive plate (130) relative to the surgical stapler (10) for firing the surgical stapler.

2. The surgical stapling system according to claim 1, wherein the auxiliary device (100) defines a longitudinal axis, the mounting bracket (110) positioned to rotate about the longitudinal axis to enable the surgical stapler (10) to rotate about the drive plate (130).

3. The surgical stapling system according to claim 2, wherein the mounting bracket (110) is positioned to rotate in a clockwise direction up to about 90 degrees about the longitudinal axis, and wherein the mounting bracket (110) is positioned to rotate in a counterclockwise direction up to about 90 degrees about the longitudinal axis.

4. The surgical stapling system according to claim 2 or claim 3, wherein the drive plate (130) defines a slot (131) in which the firing actuator (18) is received.

5. The surgical stapling system according to claim 4, wherein the slot (131) is configured to slidably support the firing actuator (18) as the surgical stapler (10) is rotated about the auxiliary device (100).

6. The surgical stapling system according to any preceding claim, wherein the drive plate (130) is axially movable along the longitudinal axis.

7. The surgical stapling system according to claim 6, wherein the auxiliary device (100) further includes a stationary handle (170), the firing lever (150) movable relative to the stationary handle.

8. The surgical stapling system according to claim 7, further comprising a pawl (157), and wherein the rack (132) includes a plurality of teeth (134), the pawl (157) configured to mechanically engage with the plurality of teeth (134) to move the rack (132) relative to the stationary handle (170) as the firing lever (150) moves relative to the stationary handle (170).

9. The surgical stapling system according to any preceding claim, wherein the drive plate includes a retraction handle (140), which when pulled, draws the drive plate (130) toward an unfired position.

## Patentansprüche

1. Chirurgisches Klammernahtsystem, umfassend:
ein chirurgische Klammernahtvorrichtung (10), die einen Endeffektor und einen Auslöseaktuator (18) aufweist, der zum Auslösen des Endeffektors ausgestaltet ist,
**dadurch gekennzeichnet, dass** das chirurgische Klammernahtsystem ferner eine Hilfsvorrichtung (100) umfasst, die Folgendes aufweist:
eine lösbar an die chirurgische Klammernahtvorrichtung (10) gekoppelte Montagehalterung (110),
eine an den Auslöseaktuator (18) der chirurgischen Klammernahtvorrichtung (10) gekoppelte Antriebsplatte (130),
eine mechanisch an die Antriebsplatte (130) gekoppelte Zahnstange (132) und
einen Auslösehebel (150), der mechanisch an die Zahnstange (132) gekoppelt und schwenkbar ist, um die Antriebsplatte (130) relativ zu der chirurgischen Klammernahtvorrichtung (10) zum Auslösen der chirurgischen Klammernahtvorrichtung zu bewegen.

2. Chirurgisches Klammernahtsystem nach Anspruch 1, wobei die Hilfsvorrichtung (100) eine Längsachse definiert, wobei die Montagehalterung (110) so positioniert ist, dass sie sich um die Längsachse dreht, damit sich die chirurgische Klammernahtvorrichtung (10) um die Antriebsplatte (130) drehen kann.

3. Chirurgisches Klammernahtsystem nach Anspruch 2, wobei die Montagehalterung (110) so positioniert ist, dass sie sich im Uhrzeigersinn bis zu etwa 90 Grad um die Längsachse dreht, und wobei die Montagehalterung (110) so positioniert ist, dass sie sich entgegen dem Uhrzeigersinn bis zu etwa 90 Grad um die Längsachse dreht.

4. Chirurgisches Klammernahtsystem nach Anspruch 2 oder Anspruch 3, wobei die Antriebsplatte (130) einen Schlitz (131) definiert, in dem der Auslöseaktuator (18) aufgenommen ist.

5. Chirurgisches Klammernahtsystem nach Anspruch 4, wobei der Schlitz (131) dazu ausgestaltet ist, den Auslöseaktuator (18) gleitend zu stützen, wenn die chirurgische Klammernahtvorrichtung (10) um die Hilfsvorrichtung (100) gedreht wird.

6. Chirurgisches Klammernahtsystem nach einem der vorhergehenden Ansprüche, wobei die Antriebsplatte (130) entlang der Längsachse axial beweglich ist.

7. Chirurgisches Klammernahtsystem nach Anspruch 6, wobei die Hilfsvorrichtung (100) ferner einen feststehenden Griff (170) aufweist, wobei der Auslösehebel (150) relativ zu dem feststehenden Griff beweglich ist.

8. Chirurgisches Klammernahtsystem nach Anspruch 7, ferner umfassend eine Klinke (157), und wobei die Zahnstange (132) eine Vielzahl von Zähnen (134) aufweist, wobei die Klinke (157) dazu ausgestaltet ist, mechanisch mit der Vielzahl von Zähnen (134) in Eingriff zu kommen, um die Zahnstange (132) relativ zu dem feststehenden Griff (170) zu bewegen, während sich der Auslösehebel (150) relativ zu dem feststehenden Griff (170) bewegt.

9. Chirurgisches Klammernahtsystem nach einem der vorhergehenden Ansprüche, wobei die Antriebsplatte einen Rückziehgriff (140) aufweist, der die Antriebsplatte (130) zu einer unausgelösten Position hin zieht, wenn an ihm gezogen wird.

## Revendications

1. Système d'agrafage chirurgical comprenant :
une agrafeuse chirurgicale (10) comportant un effecteur terminal et un actionneur de déclenchement (18) configuré pour déclencher l'effecteur terminal ;
**caractérisé en ce que** le système d'agrafage chirurgical comprend en outre un dispositif auxiliaire (100) comportant :
un support de montage (110) accouplé de manière amovible à l'agrafeuse chirurgicale (10) ;
une plaque d'entraînement (130) accouplée à l'actionneur de déclenchement (18) de l'agrafeuse chirurgicale (10) ;
une crémaillère (132) accouplée mécaniquement à la plaque d'entraînement (130) ; et
un levier de déclenchement (150) accouplé mécaniquement à la crémaillère (132) et pouvant pivoter pour déplacer la plaque d'entraînement (130) par rapport à l'agrafeuse chirurgicale (10) afin de déclencher l'agrafeuse chirurgicale.

2. Système d'agrafage chirurgical selon la revendication 1, dans lequel le dispositif auxiliaire (100) définit un axe longitudinal, le support de montage (110) étant positionné pour tourner autour de l'axe longitudinal afin de permettre à l'agrafeuse chirurgicale (10) de tourner autour de la plaque d'entraînement (130).

3. Système d'agrafage chirurgical selon la revendication 2, dans lequel le support de montage (110) est positionné pour tourner dans le sens des aiguilles d'une montre jusqu'à environ 90 degrés autour de l'axe longitudinal, et dans lequel le support de montage (110) est positionné pour tourner dans le sens inverse des aiguilles d'une montre jusqu'à environ 90 degrés autour de l'axe longitudinal.

4. Système d'agrafage chirurgical selon la revendication 2 ou la revendication 3, dans lequel la plaque d'entraînement (130) définit une fente (131) dans laquelle est reçu l'actionneur de déclenchement (18).

5. Système d'agrafage chirurgical selon la revendication 4, dans lequel la fente (131) est configurée pour supporter de manière coulissante l'actionneur de déclenchement (18) à mesure que l'agrafeuse chirurgicale (10) tourne autour du dispositif auxiliaire (100).

6. Système d'agrafage chirurgical selon l'une quelconque des revendications précédentes, dans lequel la plaque d'entraînement (130) est mobile axialement le long de l'axe longitudinal.

7. Système d'agrafage chirurgical selon la revendication 6, dans lequel le dispositif auxiliaire (100) comporte en outre une poignée fixe (170), le levier de déclenchement (150) étant mobile par rapport à la poignée fixe.

8. Système d'agrafage chirurgical selon la revendication 7, comprenant en outre un cliquet (157), et dans lequel la crémaillère (132) comporte une pluralité de dents (134), le cliquet (157) étant configuré pour venir mécaniquement en prise avec la pluralité de dents (134) pour déplacer la crémaillère (132) par rapport à la poignée fixe (170) à mesure que le levier de déclenchement (150) se déplace par rapport à la poignée fixe (170).

9. Système d'agrafage chirurgical selon une quelconque revendication précédente, dans lequel la plaque d'entraînement comporte une poignée de rétraction (140) qui, lorsqu'elle est tirée, tire la plaque d'entraînement (130) vers unae position non déclenchée.
